# FASCICULE DE BREVET EUROPEEN

(11) **EP 1 741 386 B1**
(45) Date de publication et mention de la délivrance du brevet: **22.04.2009**
(21) Numéro de dépôt: 06291001.3
(22) Date de dépôt: 21.06.2006
(51) Int. Cl.: A61B 5/08, A61N 1/37, A61B 5/11, A61N 1/36

(54) **Dispositif médical implantable actif comprenant des moyens de détection d'apnées ou hypopnées**
Implantierbare medizinische Vorrichtung mit Mittel zur Detektion von Schlafapnoe oder Hypopneen
Implantable medical device with means for detection of sleep apnea or hypopnea

(30) Priorité: 05.07.2005 FR 0507121; 14.03.2006 FR 0602202
(43) Date de publication de la demande: 10.01.2007
(73) Titulaire: ELA MEDICAL, 92541 Montrouge (FR)
(72) Inventeur: Vincent, Elodie, 92330 Sceaux (FR); Vitali, Luca, 10019 Strambino (IT)
(74) Mandataire: Dupuis-Latour, Dominique

(56) Documents cités:
- EP-A- 0 970 713
- EP-A- 1 336 422
- EP-A- 1 413 330
- EP-A- 1 433 496
- EP-A- 1 533 001
- EP-A- 1 537 894

## Description

L'invention concerne les "dispositifs médicaux implantables actifs" tels que définis par la directive 90/385/CE du 20 juin 1990 du Conseil des communautés européennes, notamment les stimulateurs cardiaques, dispositifs de resynchronisation, cardioverteurs et/ou défibrillateurs destinés au traitement des troubles du rythme cardiaque, ou encore les implants actifs à visée purement diagnostique.

Elle concerne plus précisément ceux de ces dispositifs implantables qui sont dotés d'une fonction de détection des troubles du rythme respiratoire survenant pendant une phase de sommeil chez le patient porteur de l'appareil implanté.

De façon générale, la pathologie respiratoire connue sous le nom de "syndrome d'apnée du sommeil" (SAS) est caractérisée par la survenue fréquente (au moins 10 à 20 fois par heure) d'apnées pendant une phase de sommeil du patient, une "apnée" (ou pause respiratoire) étant définie comme un arrêt temporaire de la fonction respiratoire de durée supérieure à 10 secondes. Elle peut également être caractérisée par la survenue d'hypopnées dans les mêmes conditions, une "hypopnée" étant définie comme une décroissance importante (mais sans interruption) du débit respiratoire, typiquement une décroissance de plus de 50 % par rapport à une moyenne de référence antérieure.

Face à cette pathologie, qui atteint plus de 4% de la population et plus de 50% des patients souffrant d'insuffisance cardiaque, le système nerveux autonome s'adapte mais avec un effet délétère sur le sommeil, l'interruption ou la réduction du débit respiratoire entraînant une diminution de la concentration en oxygène du sang ainsi que des micro-réveils inconscients. II s'ensuit en phase d'éveil une somnolence diurne avec perte d'attention et un accroissement des risques d'accident de la route. Par ailleurs, la réponse adaptative physiologique, puis pathologique, de certains organes dont le coeur et l'appareil circulatoire entraîne une plus grande incidence de troubles tels qu'hypertension artérielle, arythmies ventriculaires, infarctus myocardique et insuffisance cardiaque.

On connaît diverses techniques pour détecter les troubles respiratoires du sommeil au moyen d'un dispositif implantable.

Ainsi, le EP-A-0 970 713 (ELA Medical) divulgue un dispositif diagnostiquant la survenue d'une apnée à partir du signal de ventilation-minute (signal VE, dit également signal MV), paramètre à prépondérance physiologique généralement obtenu par une mesure d'impédance transthoracique donnant en continu une indication du rythme respiratoire du patient.

Cette mesure de ventilation-minute est réalisée par injection d'impulsions de courant entre deux électrodes disposées dans la cage thoracique, ou bien entre le boîtier du dispositif implanté et une électrode, par exemple une électrode de stimulation. Les variations d'impédance reproduisent les variations du volume thoracique, avec des pics d'impédance lors de l'inspiration, lorsque les poumons sont remplis d'air, et une impédance décroissante lors de la phase expiratoire.

Il a toutefois été constaté en pratique, lors d'études cliniques, que cette technique de mesure d'activité respiratoire enregistrant les variations de volume pulmonaire au niveau thoracique peut être leurrée dans certaines circonstances, conduisant à des risques de faux positifs et de faux négatifs susceptibles de perturber l'interprétation des signaux par le dispositif et conduire ainsi à un diagnostic erroné.

Ainsi, l'impédance transthoracique varie en fonction de la résistivité des tissus au moment de l'injection de l'impulsion de courant ; comme cette résistivité dépend essentiellement de la quantité d'air dans les poumons et de la quantité de sang dans les cavités cardiaques, le signal d'impédance recueilli est modulé à la fois par la respiration et par la fréquence cardiaque. L'impédance est également modulée par les variations de distance entre l'électrode de mesure et le boîtier du dispositif, distance qui varie cycliquement en fonction des battements cardiaques.

D'autre part, à la composante respiratoire du signal (variation dynamique, seule significative) s'ajoute une composante statique, liée à l'impédance des tissus constatée dans une position corporelle stable et en l'absence de toute respiration et de tout battement cardiaque.

Ainsi, l'impédance transthoracique peut être modifiée par les mouvements du patient, ou bien varier sous l'effet des contractions du diaphragme au cours d'une apnée obstructive. Ces phénomènes produisent des artéfacts perturbant le système et peuvent conduire à une détection erronée de cycles respiratoires particulièrement amples ou rapides, ou au contraire d'amplitude faible et/ou de périodes longues, pouvant conduire à un faux positif.

Un autre type d'artéfact peut résulter de la présence dans le signal d'impédance d'une composante liée aux battements cardiaques. En effet, dans certaines circonstances (par exemple en situation à la fois de bradycardie et d'hyperventilation), la fréquence respiratoire et la fréquence cardiaque peuvent devenir suffisamment proches pour que les battements cardiaques influencent de façon significative le signal d'impédance. Le rythme cardiaque peut se trouver alors interprété à tort comme un rythme respiratoire, risquant ainsi de masquer la présence d'une apnée ou d'une hypopnée (faux négatif, au moment au moment où un véritable évènement pathologique respiratoire survient).

Idéalement, pour diagnostiquer un trouble respiratoire du sommeil en s'affranchissant des inconvénients de la mesure d'impédance transthoracique, le meilleur critère serait une mesure de la saturation en oxygène dans le sang, le diagnostic de SAS n'étant confirmé qu'en cas de désaturation avérée et significative.

En effet, les patients souffrant d'apnées ou hypopnées présentent des variations cycliques de la fréquence cardiaque et de la pression artérielle : au moment du micro-réveil et de la reprise ventilatoire qui font suite à l'apnée se produit en effet une réaction adrénergique qui induit une tachycardie et une augmentation du débit cardiaque qui compense ainsi l'hypoxémie induite par l'apnée de sorte que, par réaction, le sang est ainsi maintenu au même niveau de saturation en oxygène.

Mais cette mesure directe est difficile à réaliser de manière simple et permanente dans le contexte d'un dispositif implanté.

Une autre approche du diagnostic des pathologies de l'activité respiratoire par un dispositif implanté est basée sur l'analyse des pics d'accélération endocardiaque, qui est un paramètre reflétant de façon non artéfactée et avec un très faible temps de réponse les variations de la contractilité du myocarde.

Les EP-A-1 433 496 et EP-A-1 413 330 divulguent de tels dispositifs de diagnostic des troubles respiratoires du sommeil pourvus de moyens de mesure et d'analyse de l'accélération endocardiaque.

Afin d'obtenir une meilleure détection des évènements respiratoires pathologiques, ce signal d'accélération endocardiaque peut être analysé de manière croisée ― mais de façon subsidiaire ― avec d'autres signaux physiologiques tels que la fréquence cardiaque, la ventilation-minute et/ou l'activité du patient, donnée par exemple par un capteur d'accélération.

Le dispositif de l'invention est un dispositif médical implantable actif doté d'une fonction de détection des apnées ou hypopnées respiratoires survenant chez le patient porteur de l'appareil implanté, du type général divulgué par le EP-A-1 433 496 précité, correspondant au préambule de la revendication 1. La partie caractérisante de cette revendication 1 énonce les éléments spécifiques de l'invention. Les sous-revendications visent des formes de mise en oeuvre particulières avantageuses.

On va maintenant décrire plus en détail l'invention, en référence aux figures annexées.

La figure 1 est un chronogramme montrant les variations au cours de trois cycles cardiaques successifs de l'accélération endocavitaire ainsi que de l'électrogramme et de l'électrocardiogramme de surface correspondants.

La figure 2 illustre les variations du rapport entre moyenne à court terme et moyenne à long terme de l'accélération endocavitaire au cours d'un épisode d'apnée typique.

En ce qui concerne ses aspects logiciels, l'invention peut être mise en oeuvre par une programmation appropriée du logiciel de commande d'un stimulateur connu, par exemple de type stimulateur cardiaque ou défibrillateur/cardioverteur, comprenant des moyens d'acquisition d'un signal fourni par des sondes endocavitaires et/ou un ou plusieurs capteurs implantés.

L'invention peut notamment être appliquée aux dispositifs implantables commercialisés par ELA Médical, Montrouge, France tels que les appareils *Symphony* et *ELA Rhapsody.*

Il s'agit de dispositifs à microprocesseur programmable comportant des circuits pour recevoir, mettre en forme et traiter des signaux électriques recueillis par des électrodes implantées, et délivrer des impulsions de stimulation à ces électrodes. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail.

On sait recueillir un signal d'accélération endocardiaque, comme cela est par exemple exposé dans le EP-A-0 515 319 (Sorin Biomedica Cardio SpA) qui décrit une sonde endocavitaire pourvue d'une électrode distale de stimulation implantée au fond d'une cavité cardiaque (ventricule gauche ou droit, voire même dans l'oreillette), intégrant en outre un micro-accéléromètre permettant de mesurer l'accélération endocardiaque.

Le signal d'accélération endocardiaque ainsi mesuré au cours d'un cycle cardiaque forme, entre autres, deux pics correspondant aux deux bruits majeurs qu'il est possible de reconnaître dans chaque cycle d'un coeur sain. Le EP-A-0 655 260 (Sorin Biomedica Cardio SpA) décrit une manière de traiter le signal d'accélération endocavitaire délivré par le capteur situé en bout de sonde pour en dériver, notamment, ces deux valeurs de pic d'accélération endocardiaque, utiles en particulier pour la détection de troubles cardiaques et le déclenchement ou non d'une thérapie de défibrillation.

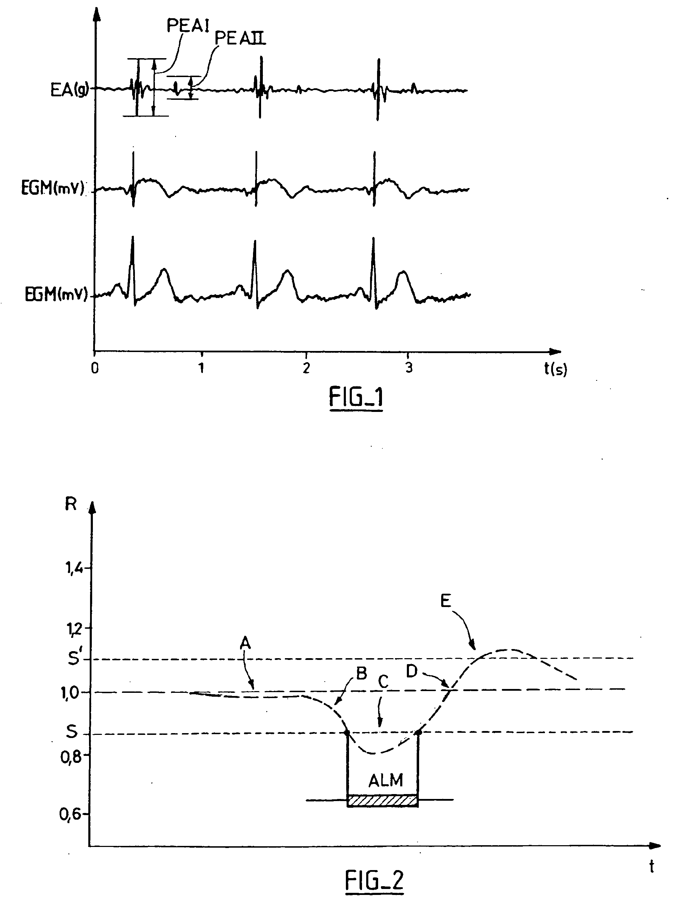

Sur la figure 1 on a représenté (courbe du haut), les variations de l'accélération endocardiaque (EA), mesurée par un capteur tel que celui décrit dans le EP-A-0 515 319 précité, intégré à une tête de sonde endocavitaire placée au fond du ventricule. On a également illustré sur cette figure les tracés de l'électrogramme (EGM), c'est-à-dire du signal électrique recueilli par l'électrode distale de ce même capteur, et d'un électrocardiogramme de surface (ECG) correspondant, au cours de trois cycles cardiaques consécutifs.

Comme on peut le voir, l'accélération endocardiaque passe par deux pics successifs dont l'amplitude peut être déterminée par un traitement approprié du signal délivré par le capteur d'accélération, comme décrit dans le EP-A-0 655 260 précité. Par "pic" on entendra la valeur maximale crête-à-crête du signal d'accélération séparant les deux extrema, positif et négatif, correspondant aux écarts PEA I et PEA II indiqués sur le chronogramme de la figure.

Plus précisément, le premier pic d'accélération endocardiaque ("PEA I") correspond à la fermeture des valvules mitrale et tricuspide, au début de la phase de contraction ventriculaire isovolumique (systole). Les variations de ce premier pic sont étroitement liés aux variations de la pression dans le ventricule (l'amplitude du pic PEA I étant, plus précisément, corrélée au maximum positif de la variation de pression dP/dt dans le ventricule gauche) et peuvent donc constituer un paramètre représentatif de la contractilité du myocarde, elle-même liée au niveau d'activité du système sympathique.

Le second pic d'accélération endocardiaque ("PEA II"), quant à lui, correspond à la fermeture des valvules aortique et pulmonaire, au moment de la phase de relaxation ventriculaire isovolumique. Ce second pic, qui est produit par la décélération brusque de la masse sanguine en mouvement dans l'aorte, constitue un paramètre représentatif de la pression sanguine périphérique au début de la diastole. Il constitue également un paramètre-clé du processus physiologique conduisant à la survenue d'une syncope vasovagale.

Les valeurs de pics PEA I et/ou PEA II sont relevées au cours de cycles successifs, ainsi qu'éventuellement la fréquence cardiaque.

Ces signaux peuvent être traités de diverses manières.

Une première technique consiste à déterminer, cycle à cycle, les valeurs absolues que prennent ces paramètres et à fixer des seuils de déclenchement d'alarme ― ou, de préférence, déterminer une valeur moyennée de ces paramètres sur un nombre prédéterminé de cycles, pour éviter les influences de la variabilité cycle-à-cycle (dispersion des mesures) et celles des évènements brefs non significatifs.

Pour décider de la présence ou non d'une apnée ou hypopnée, un ou plusieurs seuils sont fixés, et chacun des paramètres PEA I ou PEA II (ou une combinaison de ces deux paramètres) est comparé à un seuil prédéterminé. Le résultat de la comparaison peut être combiné de diverses manières avec le résultat de comparaisons semblables d'autres paramètres (notamment la fréquence cardiaque) pour produire un signal de sortie à deux états, l'un des états étant associé à une situation normale et l'autre état étant associé à une alerte d'apnée ou hypopnée.

Pour améliorer la spécificité de la détection, et notamment pour tenir compte des différences de valeur de base des paramètres PEA d'un individu à l'autre, il peut être avantageux d'analyser les variations de ces paramètres plutôt que leurs valeurs absolues.

Une manière de procéder consiste à analyser la différence entre une moyenne à court terme et une moyenne à long terme du même paramètre. Si ce paramètre varie peu, la différence sera faible et les deux valeurs finiront par coïncider. En revanche, dès que le paramètre deviendra instable, la moyenne à court terme va suivre les variations du paramètre plus rapidement que la moyenne à long terme. La différence entre les deux moyennes ne sera plus nulle ou quasi-nulle, mais prendra une valeur positive (en cas d'augmentation du paramètre) ou négative (en cas de diminution), la valeur absolue de cet écart dépendant du paramètre analysé et de la vitesse de variation de celui-ci.

Il est également possible de suivre non pas la différence, mais le ratio entre moyenne à court terme et moyenne à long terme des paramètres PEA I et/ou PEA II.

Ainsi, en référence à la figure 2, un exemple d'algorithme de détection d'apnée consiste à calculer une moyenne mobile à court terme des valeurs cycle à cycle du paramètre PEA I, par exemple sur quatre cycles consécutifs. Simultanément, une moyenne mobile à long terme, par exemple sur 500 cycles, de ce même paramètre est calculée et mise à jour. Les deux moyennes sont alors comparées pour donner un ratio R, où R = moyenne à court terme/moyenne à long terme.

En régime stationnaire, le ratio R présente une valeur à peu près constante et voisine de l'unité.

En revanche, lors d'un épisode d'apnée, la diminution de la saturation en oxygène dans le sang induit une réduction de la contractilité cardiaque qui se traduit par une diminution correspondante de l'amplitude du paramètre PEA I pendant plusieurs battements consécutifs, et donc une diminution corrélative de la valeur du ratio R.

Cette situation est illustrée sur la figure 2, où A indique le début de l'épisode d'apnée et B correspond au moment où l'accélération endocardiaque chute brutalement suite à la réduction de la contractilité.

II est possible de déclencher une alarme en définissant pour le ratio R un seuil approprié S, par exemple S = 0,85 ou S = 0,9, et en comparant la valeur courante du ratio R à ce seuil. Chaque fois que la ratio R tombe en dessous de ce seuil (situation illustrée en C sur la figure 2), une alarme (ALM) est déclenchée.

Cette alarme peut être déclenchée immédiatement au moment du franchissement du seuil, ou bien de manière conditionnelle, par exemple seulement si le ratio R descend sous le seuil pendant une durée minimale prédéterminée (par exemple 5 secondes) ou un nombre de cycles prédéterminé (par exemple 5 cycles).

L'activation de l'alarme indique ainsi un épisode de dépression soudaine de la contractilité cardiaque, qui est associé à un épisode d'apnée (ou d'hypopnée). En réponse, il est notamment possible de déclencher l'inscription d'informations de diagnostic dans une mémoire du dispositif (marqueur de survenue d'une apnée, horodatage de cette apnée, durée de l'alarme, ...).

L'alarme peut également être utilisée pour déclencher une thérapie appropriée. Par exemple, le dispositif implanté peut augmenter la fréquence de stimulation afin de maintenir la fonction cardiaque à un niveau approprié permettant une irrigation satisfaisante des tissus, cette dernière étant compromise lors des épisodes d'apnée du fait d'un débit cardiaque réduit du fait de l'activité insuffisante du système sympathique.

La thérapie déclenchée par l'alarme peut être prolongée jusqu'à ce que le ratio R revienne à son niveau de base, c'est-à-dire à environ R = 1 (la référence D sur la figure 2 indique la fin de l'épisode d'apnée).

En variante, la thérapie peut être programmée indépendamment pour une durée fixe, cette durée programmable étant choisie de manière à se prolonger nettement au-delà d'un épisode apnéique isolé, afin d'assurer un soutien de la fonction cardiaque pendant une période plus longue.

Par ailleurs on observe généralement après la fin de l'épisode d'apnée un phénomène de rebond, correspondant à une augmentation transitoire de la contractilité cardiaque (ce rebond est illustré en E sur la figure 2). Plus précisément, à la fin d'un épisode d'apnée survient souvent un micro-réveil au cours duquel le système sympathique est activé en réaction aux évènements antérieurs ; cette réaction prend fin spontanément après un certain nombre de cycles cardiaques, le ratio R retournant progressivement à sa valeur de base voisine de l'unité.

Ce phénomène de rebond peut être détecté et utilisé pour confirmer la survenue de l'épisode d'apnée. À cet effet, on définit un second seuil S' de valeur supérieure à l'unité, par exemple S' = 1,1 et on détecte le franchissement de ce second seuil S' après détection d'un épisode d'apnée. On notera que le franchissement du seuil S' révèle seulement une réaction positive du système sympathico-vagal à l'état anormal induit par l'apnée, réaction qui n'est pas en soi pathologique et ne doit pas déclencher d'alarme et ni de thérapie particulière.

En revanche, la détection conjointe (i) d'une diminution du PEA révélant une réduction de la contractilité, détectée par le franchissement du premier seuil S (ratio R < 0,85), suivie (ii) d'une augmentation du PEA, détectée par le franchissement du second seuil (ratio R > 1,1), et ce (iii) dans un intervalle de temps prédéterminé, par exemple dans un laps de temps d'une minute, est un indice fort d'un état non physiologique rencontré typiquement chez des patients souffrant de troubles du sommeil.

La détection d'un tel profil typique de variation du PEA peut être utilisée pour calculer un indice spécifique, permettant de diagnostiquer chez le sujet la présence ou non d'une instabilité du système sympathico-vagal.

Ce diagnostic peut permettre d'optimiser les paramètres spécifiques de la thérapie à lui appliquer.

Par exemple, la détection d'un profil récurrent de diminution/augmentation du PEA peut être associée à un niveau élevé de gravité des troubles du patient, conduisant à choisir une thérapie plus forte, par exemple une fréquence de stimulation supérieure et/ou une réponse plus rapide lorsqu'une diminution du PEA est détectée. Le paramétrage de la thérapie peut être opéré soit manuellement par le praticien après diagnostic, soit automatiquement, de manière adaptative, par le dispositif implanté.

Inversement, une meilleure stabilité du PEA peut être associée à une condition moins sévère du patient, conduisant à programmer une thérapie plus modérée.

Il est possible de combiner cette analyse du PEA à une analyse parallèle de la fréquence cardiaque, pour améliorer la sensibilité et la spécificité de la détection et de la classification des épisodes d'apnée.

Par ailleurs, il est possible de prendre en compte le paramètre PEA II en variante ou en complément du PEA I pour la détection des apnées comme dans l'exemple qui précède. Le PEA II peut être utilisé comme indicateur d'une contractilité réduite, dans la mesure où il est corrélé aux variations de la pression sanguine. Plus précisément, une contractilité réduite combinée à un ralentissement du rythme cardiaque provoque une diminution de la pression sanguine, qui se traduit par une réduction de l'amplitude du PEA II. L'analyse de ce paramètre permet ainsi de confirmer les épisodes d'apnée, et de déclencher et/ou modifier la thérapie spécifique appliquée pour traiter ces épisodes d'apnée ou d'hypopnée.

D'autres types d'analyses, plus complexes, peuvent également être mises en oeuvre pour améliorer encore la qualité du procédé de détection, par exemple des techniques de corrélation, d'analyse de morphologie du signal, d'analyse fréquentielle, d'analyse par ondelettes, etc.

Il est également possible d'utiliser un processus de type "machine à états", où les résultats des comparaisons aux divers seuils sont appliqués à un système à transition d'états et à mémoire, qui prend la décision de déclencher une alerte d'apnée ou hypopnée en fonction d'un schéma d'évolution plus complexe.

Le procédé de détection peut également prendre en compte non seulement les paramètres PEA I et/ou PEA II, mais également d'autres paramètres tels que la fréquence cardiaque, ou encore des signaux délivrés par, un capteur de ventilation-minute ou encore un capteur d'activité.

En effet, la fréquence cardiaque décroît au début de l'évènement respiratoire et augmente au moment du micro-réveil qui suit l'évènement, ou bien présente une variabilité caractéristique pendant la survenue de l'évènement.

La ventilation, quant à elle, présente un arrêt du flux respiratoire pendant au moins dix secondes et/ou une réduction d'au moins 50% de ce flux pendant au moins dix secondes.

Enfin, le diagnostic ne présente d'intérêt que lorsque le patient est au repos, car les variations d'activité respiratoire survenant pendant une phase d'éveil ne sont normalement pas pathologiques. La technique la plus simple pour détecter les phases de sommeil du patient consiste à utiliser une horloge interne du dispositif, commutant un indicateur à heures fixes. Il est également possible, comme enseigné par le EP-A-0 719 568 (Ela Médical), d'opérer la discrimination entre éveil et sommeil par analyse du signal de ventilation-minute : en effet, la variation circadienne de la fréquence et de l'amplitude des cycles respiratoires successifs du patient est bien reproduite par ce signal, et un calcul de la ventilation moyenne sur 24 h permet d'opérer une discrimination satisfaisante entre une ventilation d'éveil et une ventilation de sommeil. On peut également utiliser un capteur d'activité, typiquement un accéléromètre ("capteur G"), dont le signal permet de détecter les mouvements du patient ; l'information de ce type de capteur n'est en elle-même pas très spécifique des phases d'éveil et de sommeil, mais on sait combiner les signaux délivrés par un capteur G et un capteur MV pour en déduire des informations signifiantes, comme cela est décrit par exemple dans les EP-A-0 750 920 et EP-A-0 770 407, tous deux au nom d'ELA Médical, auxquels on pourra se référer pour de plus amples détails.

L'implant peut également comprendre des moyens permettant non seulement de diagnostiquer la survenue d'un SAS, mais également de caractériser plus précisément certains évènements tels qu'apnée, hypopnée ou pause respiratoire, ou des profils respiratoires particuliers, tels que des profils de type Cheyne-Stokes. On pourra se référer à cet égard aux EP-A-0 970 713, EP-A-1 336 422 et EP-A-1 295 623, tous trois au nom d'ELA Medical.

On peut en outre prévoir que le système soit auto-adaptatif, c'est-à-dire qu'il puisse s'adapter à des variations sur le long terme, afin d'améliorer ultérieurement la spécificité du système de détection.

Par ailleurs, les signaux d'accélération endocardiaque et/ou les valeurs des différents paramètres PEA peuvent être conservés dans une mémoire de l'implant, simultanément à d'autres signaux ou marqueurs. Cette mémorisation peut être déclenchée selon certaines conditions programmées ou sur détection de certains états (sommeil) ou évènements (survenue d'un épisode apnéique). Les signaux mémorisés pourront être ultérieurement visualisés par un praticien équipé d'un programmateur permettant de lire le contenu de la mémoire de l'implant. Il sera également possible, à ce stade, d'analyser le signal PEA afin d'en extraire certains paramètres qui ne sont pas déterminables en temps réel, tels que la variabilité sinusale, ou pour simuler l'application d'algorithmes de détection afin de choisir les plus approprié au cas du patient.

Enfin, le dispositif de l'invention peut être employé soit à des fins purement diagnostiques, soit pour appliquer une thérapie appropriée en cas de survenue d'apnées ou d'hypopnées détectées. Ainsi, de manière en elle-même connue, si une apnée survient pendant une phase de sommeil du patient, le dispositif peut, si certains autres critères sont remplis, délivrer une stimulation cardiaque à une fréquence légèrement supérieure au rythme sinusal naturel du patient (technique dite d"'*overdriving*"), ceci afin d'augmenter le débit sanguin afin de réduire l'incidence de la désaturation en oxygène consécutive au SAS. Ces techniques sont notamment décrites dans les EP-A-0 970 713 et EP-A-1 413 330 précités, auxquels on pourra se référer pour de plus amples détails.

## Revendications

1. Un dispositif médical implantable actif, notamment un dispositif de stimulation, resynchronisation, défibrillation et/ou cardioversion, ou un implant actif à visée diagnostique,
ce dispositif comprenant des moyens de détection de la survenue d'apnées ou d'hypopnées comportant :
- des moyens de recueil de l'accélération endocardiaque (EA) du patient, et
- des moyens d'analyse, aptes à :
· déterminer au moins un paramètre fonction de l'un et/ou de l'autre de deux pics de l'accélération endocardiaque au cours d'un cycle donné, ces deux pics comprenant un premier pic (PEA I) lors de la phase de contraction ventriculaire isovolumique et un second pic (PEA II) lors de la phase de relaxation ventriculaire isovolumique,
· comparer ledit au moins un paramètre à un premier seuil prédéterminé (S), et
· délivrer conditionnellement un signal d'alerte (ALM) d'apnée ou d'hypopnée au franchissement de ce seuil,
dispositif **caractérisé en ce que :**
- ledit au moins un paramètre est fonction d'un ratio (R) entre une moyenne à long terme et une moyenne à court terme des valeurs de pic(s) d'accélération endocardiaque relevées au cours d'une pluralité de cycles successifs,
- ce ratio (R) est comparé audit premier seuil (S) pour délivrer ledit signal d'alerte (ALM) au franchissement de ce seuil, et
- les moyens d'analyse comprennent en outre des moyens aptes, après franchissement dudit premier seuil (S), à :
· détecter un franchissement inverse de ce premier seuil suivi du franchissement d'un second seuil (S'), supérieur au premier, et
· délivrer un signal de confirmation d'épisode d'apnée ou d'hypopnée au franchissement de ce second seuil (S').

2. Le dispositif de la revendication 1, dans lequel les moyens d'analyse sont aptes à délivrer ledit signal d'alerte après franchissement dudit premier seuil seulement si ce premier seuil reste franchi pendant une durée minimale prédéterminée, ou pendant un nombre minimal prédéterminé de cycles cardiaques.

3. Le dispositif de la revendication 1, dans lequel les moyens d'analyse sont aptes à délivrer ledit signal de confirmation d'épisode d'apnée ou d'hypopnée seulement si le franchissement dudit second seuil intervient au cours d'une durée maximale prédéterminée, ou pendant un nombre maximal prédéterminé de cycles cardiaques, après ledit franchissement inverse du premier seuil.

4. Le dispositif de la revendication 1, dans lequel les moyens d'analyse comprennent une machine à états ou réseau de neurones apte à comparer une pluralité desdits paramètres à une pluralité de seuils prédéterminés, à détecter le franchissement des différents seuils, à analyser la séquence de ces franchissements et à délivrer ledit signal d'alerte sur détection d'une ou plusieurs séquence(s) de franchissement prédéterminées.

5. Le dispositif de la revendication 1, dans lequel les moyens d'analyse comprennent des moyens aptes à appliquer à l'accélération endocardiaque un traitement d'autocorrélation, d'analyse morphologique, d'analyse fréquentielle, d'analyse par ondelettes et/ou d'analyse en composantes principales.

6. Le dispositif de la revendication 1, dans lequel :
- les moyens de détection comprennent également des moyens de recueil de la fréquence cardiaque du patient, et
- les moyens d'analyse sont des moyens aptes à délivrer conditionnellement le signal d'alerte en fonction à la fois de la fréquence cardiaque du patient et de la valeur prise par ledit au moins un paramètre.

7. Le dispositif de la revendication 1, dans lequel :
- les moyens de détection comprennent également des moyens de recueil de l'activité respiratoire, aptes à délivrer un signal d'activité ventilatoire du patient, et
- les moyens d'analyse sont des moyens aptes à délivrer conditionnellement le signal d'alerte en fonction à la fois du signal d'activité ventilatoire du patient et de la valeur prise par ledit au moins un paramètre.

8. Le dispositif de la revendication 1, dans lequel :
- les moyens de détection comprennent également des moyens de détection d'au moins une phase d'effort et/ou de sommeil, aptes à délivrer un signal d'état du patient, et
- les moyens d'analyse sont des moyens aptes à délivrer conditionnellement le signal d'alerte en fonction à la fois du signal d'état du patient et de la valeur prise par ledit au moins un paramètre.

9. Le dispositif de la revendication 1, dans lequel au moins un autre paramètre est fonction :
(i) d'une moyenne, et/ou
(ii) de la variation, et/ou
(iii) d'une différence, ou d'un ratio, entre une moyenne à long terme et une moyenne à court terme,
des valeurs de pic(s) d'accélération endocardiaque relevées au cours d'une pluralité de cycles successifs.

10. Le dispositif de la revendication 1, dans lequel au moins un autre paramètre est fonction de l'intervalle entre complexe QRS et au moins l'un des pics d'accélération endocardiaque, et/ou de l'intervalle entre le premier et le second pic d'accélération endocardiaque.

## Claims

1. An active implantable medical device, particularly a device for stimulation, resynchronisation, defibrillation and/or cardioversion, or an active implant for diagnostic purposes, said device comprising means for detecting the occurrence of apneas or hypoapneas comprising :
- means for acquiring the endocardial acceleration (EA) of a patient, and
- analyzing means, adapted for determining at least one parameter that is a function of the one and/or the other of two peak endocardial acceleration values during a given cycle, said two peaks comprising a first peak (PEA I) during the isovolumic ventricular contraction phase and a second peak (PEA II) during the isovolumic ventricular relaxation phase,
- comparing said at least one parameter with a first predetermined threshold (S), and
- conditionally deliver an apnea or hypoapnea alarm signal (ALM) when passing said threshold,
**characterized in that**
- said at least one parameter is a function of a ratio (R) between a long term average and a short term average of the peak endocardial acceleration value(s) observed during a plurality of successive cycles,
- wherein said ratio (R) is compared with said first threshold (S) for delivering said alarm signal (ALM) when passing said threshold, and
- the analyzing means moreover comprise means adapted for
- detecting a reverse passing of said first threshold followed by the passing of a second threshold (S') that is greater than the first one, and
- delivering a signal for confirming the apnea or hypoapnea episode when passing said second threshold (S')
after passing said first threshold (S').

2. The device according to Claim 1, wherein the analyzing means are adapted for delivering said alarm signal after passing said first threshold only if said first threshold continues to be passed during a predetermined minimum duration or during a predetermined minimum number of cardiac cycles.

3. The device according to Claim 1, wherein the analyzing means are adapted for delivering said confirmation signal of apnea or hypoapnea episode only if the passing of said second threshold occurs during a predetermined maximum duration or during a predetermined maximum number of cardiac cycles, after said reverse passing of the first threshold.

4. The device according to Claim 1, wherein the analyzing means comprise a state machine or a neuronal network adapted for comparing a plurality of said parameters with a plurality of predetermined thresholds, detecting the passing of the different thresholds, analyzing the sequence of these passings and delivering said alarm signal when detecting one or more sequence(s) of predetermined passings.

5. The device according to Claim 1, wherein the analyzing means comprise means adapted for applying to the endocardial acceleration value an autocorrelation treatment, a morphological analysis, a frequency analysis, a wavelet analysis and/or a principal component analysis.

6. The device according to Claim 1, wherein :
- the detecting means also comprise means for acquiring the patient's cardiac frequency,
- the analyzing means are means adapted for conditionally delivering the alarm signal as a function simultaneously of the patient's cardiac frequency and of the value assumed by said at least one parameter.

7. The device according to Claim 1, wherein :
- the detecting means also comprise means for acquiring the respiratory activity, adapted for delivering a patient's ventilatory activity signal ,
- the analyzing means are means adapted for conditionally delivering the alarm signal as a function simultaneously of the patient's ventilatory activity signal and of the value assumed by said at least one parameter.

8. The device according to Claim 1, wherein :
- the detecting means also comprise means for detecting at least one stress and/or sleep phase adapted for delivering a patient's condition signal ,
- the analyzing means are means adapted for conditionally delivering the alarm signal as a function simultaneously of the patient's condition signal and of the value assumed by said at least one parameter.

9. The device according to Claim 1, wherein at least one further parameter is a function
(i) of an average value, and/or
(ii) of the variation, and/or
(iii) of a difference or a ratio between a long term average and a short term average
of the values of the peak endocardial acceleration(s) acquired during a plurality of successive cycles.

10. The device according to Claim 1, wherein at least one further parameter is a function of the interval between the QRS complex and at least one of the peak endocardial acceleration values and/or of the interval between the first and the second peaks of endocardial acceleration.

## Patentansprüche

1. Eine aktive implantierbare medizinische Vorrichtung, insbesondere eine Vorrichtung zur Stimulation, Resynchronisation, Defibrillation und/oder Kardioversion oder ein aktives Implantat zur diagnostischen Absicht, wobei diese Vorrichtung Mittel zum Nachweis des Vorkommens von Apnoen bzw. Hypoapnoen umfasst, umfassend
- Mittel zur Erfassung der endokardialen Beschleunigung (EA) des Patienten,
- Analysemittel, die geeignet sind,
- wenigstens einen Parameter zu bestimmen, der von einem und/oder dem anderen der zwei Spitzenwerte während eines gegebenen Zyklus abhängt, wobei die besagten zwei Spitzenwerte einen ersten Spitzenwert (PEA I) während der isovolumischen Ventrikelkontraktionsphase und einen zweiten Spitzenwert (PEA II) während der isovolumischen Ventrikelrelaxationsphase umfassen,
- den wenigstens einen Parameter mit einer ersten vorbestimmten Schwelle (S) zu vergleichen, und
- bedingt ein Apnoe- bzw. Hypoapnoe-Alarmsignal beim Überschreiten dieser Schwelle zu liefern,
wobei die Vorrichtung **dadurch gekennzeichnet ist, dass** :
- der besagte wenigstens ein Parameter von einem Verhältnis (R) zwischen einem Langzeitmittelwert und einem Kurzzeitmittelwert des bzw. der während einer Vielzahl aufeinanderfolgenden Zyklen gemessenen Spitzenwerte der endokardialen Beschleunigung abhängt,
- dieses Verhältnis (R) wird mit der besagten ersten Schwelle (S) verglichen wird, um das besagte Alarmsignal (ALM) beim Überschreiten dieser Schwelle zu liefern,
- die Analysemittel ausserdem Mittel aufweisen, die nach dem Überschreiten der besagten ersten Schwelle (S) dazu geeignet sind :
- ein umgekehrtes Überschreiten dieser ersten Schwelle gefolgt vom Überschreiten einer Schwelle (S'), die grösser als die erste ist, nachzuweisen,
- ein Signal zur Bestätigung einer Apnoe- bzw. Hypoapnoe-Episode beim Überschreiten jener zweiten Schwelle (S') zu liefern.

2. Die Vorrichtung nach Anspruch 1, in welcher die Analysemittel dazu geeignet sind, das besagte Alarmsignal nach dem Überschreiten der ersten Schwelle zu liefern, erst wenn diese erste Schwelle während einer vorbestimmten Dauer oder während einer vorbestimmten minimale Zahl von Herzzyklen überschritten bleibt.

3. Die Vorrichtung nach Anspruch 1, in welcher die Analysemittel dazu geeignet sind, das besagte Signal zur Bestätigung einer Apnoe- bzw. Hypoapnoe-Episode zu liefern, erst wenn das Überschreiten der besagten zweiten Schwelle während einer vorbestimmten maximalen Dauer oder während einer vorbestimmten maximalen Zahl von Herzzyklen nach dem umgekehrten Überschreiten der ersten Schwelle auftritt.

4. Die Vorrichtung nach Anspruch 1, in welcher die Analysemittel einen endlichen Automat oder ein Neuronnetzwerk umfassen, die dazu geeignet ist, eine Vielzahl von besagten Parametern mit einer Vielzahl von vorbestimmten Schwellen zu vergleichen, das Überschreiten der verschiedenen Schwellen nachzuweisen, die Sequenz dieser Überschreiten zu analysieren und das besagte Signal beim Nachweis einer oder mehrerer vorbestimmten Überschreitungssequenz(en) zu analysieren.

5. Die Vorrichtung nach Anspruch 1, in welcher die Analysemittel Mittel aufweisen, die dazu geeignet sind, die endokardiale Beschleunigung durch Autokorrelation, morphologische Analyse, Frequenzanalyse, Wellchenanalyse und/oder Hauptkomponentenanalyse zu behandeln.

6. Vorrichtung nach Anspruch 1, in welcher :
- die Nachweismittel auch Mittel zur Erfassung der Herzfrequenz des Patienten aufweisen,
- die Analysemittel Mittel sind, die dazu geeignet sind, bedingt das Alarmsignal abhängig gleichzeitig von der Herzfrequenz des Patienten und dem vom besagten wenigsten einen Parameter angenommenen Wert zu liefern.

7. Vorrichtung nach Anspruch 1, in welcher :
- die Nachweismittel auch Mittel zur Erfassung der Atmungsaktivität des Patienten aufweisen, die dazu geeignet sind, ein Atmungsaktivitätssignal des Patienten zu liefern,
- die Analysemittel Mittel sind, die dazu geeignet sind, bedingt das Alarmsignal abhängig gleichzeitig vom Atmungsaktivitätssignal des Patienten und dem vom besagten wenigsten einen Parameter angenommenen Wert zu liefern.

8. Vorrichtung nach Anspruch 1, in welcher :
- die Nachweismittel auch Mittel zur Erfassung wenigstens einer Stressphase und/oder einer Schlafphase, die dazu geeignet sind, ein Zustandssignal des Patienten zu liefern,
- die Analysemittel Mittel sind, die dazu geeignet sind, bedingt das Alarmsignal abhängig gleichzeitig vom Zustandssignal des Patienten und dem vom besagten wenigsten einen Parameter angenommenen Wert zu liefern.

9. Vorrichtung nach Anspruch 1, in welcher wenigstens ein anderer Parameter
(i) von einem Mittelwert, und/oder
(ii) von der Veränderung, und/oder
(iii) von einem Unterschied oder einem Verhältnis zwischen ein Langzeitmittelwert und einem Kurzzeitmittelwert,
der während einer Vielzahl von aufeinanderfolgenden Zyklen gemessenen Spitzenwerte der endokardialen Beschleunigung(en), abhängig ist.

10. Vorrichtung nach Anspruch 1, in welcher wenigstens ein anderer Parameter vom Intervall zwischen QRS-Komplex und wenigstens einem der Spitzenwerte der endokardialen Beschleunigung und/oder vom Intervall zwischen dem ersten und dem zweiten Spitzenwerte der endokardialen Beschleunigung abhängig ist.
